# EUROPEAN PATENT SPECIFICATION

(11) **EP 0 449 941 B1**
(45) Date of publication and mention of the grant of the patent: **02.03.1994**
(21) Application number: 90901082.9
(22) Date of filing: 22.12.1989
(51) Int. Cl.: A61F 2/02, A61C 8/00, A61L 27/00

(54) **SURGICAL BARRIER**
CHIRURGISCHE GRENZSCHICHT
BARRIERE CHIRURGICALE

(30) Priority: 23.12.1988 SE 8804641
(43) Date of publication of application: 09.10.1991
(73) Proprietor: Guidor Aktiebolag, S-141 52 Huddinge (SE)
(72) Inventor: LUNDGREN, Dan, S-436 51 Hovas (SE)
(74) Representative: Ström, Tore
(86) International application number: SE8900746
(87) International publication number: WO9007308

(56) References cited:
- EP-A- 0 131 831
- WO-A-86/00517
- WO-A-86/02824
- SE-A-86 045 713
- US-A- 4 531 916
- US-A- 4 599 084

## Description

The invention relates to a barrier for guided or controlled tissue regeneration to be utilized for selective influence on the healing process during regeneration of supporting tissues adjacent to teeth and dental implants as well as during healing after periapical surgery. The barrier can also be used in connection with bone surgery to control bone fill of bone cavities resulting from cysts and malformations and of diastases following bone fractures.

The barrier of the invention comprises a shapable thin membrane, and a barrier of the this kind is disclosed in WO 86/00517.

It has not been possible previously to regenerate lost tooth supporting tissues to a clinically relevant degree. However, recent studies have shown that periodontal attachment and support structures may be regenerated (J. Gottlow, S. Nyman, J. Lindhe, T. Karring and J. Wennström, J. Clin. Perio., 13, (1986) 604-616). Given the proper conditions, cells of the periodontal ligament can be encouraged to repopulate a previously diseased tooth root surface. This concept offers the hope for renewing periodontal structures once thought to be lost permanently due to disease. The above and other studies suggest that, after periodontal surgery, a race begins among the cells from the four types of periodontal tissues; gingival epithelium, gingival connective tissue, alveolar bone and periodontal ligament to repopulate the previously diseased root surface. If uncontrolled the healing process usually results in downgrowth of cells from the gingival epithelium along the surface of the gingival connective tissue immediately lateral to the root surface thereby preventing migration of cementoblasts from the adjacent periodontal ligament to form new cementum on the denuded root surface to which new periodontal fibers can attach. Even if the gingival connective tissue, also quick to take part in the healing process, occupies the space immediately lateral to the root surface thereby hindering the epithelial cells to grow down, there will be no true new attachment between that type of tissue and the root surface but a substantial risk of root resorption (S Nyman, T Karring, J Lindhe and S Planten, J Clin. Periodontal, 7 (1980) 394-401; T Karring, F Isidor, S Nyman and J Lindhe, J clin. Periodontal 12 (1985) 51-60).

If the alveolar bone which usually regenerates slower than the gingival epithelium and connective tissue, happens to fill up parts of the space adjacent to the root surface and thereby reaches the root surface the bone will form an ancylotic union with the part of the root which is unprotected by root cementum and periodontal ligament tissue (T Karring, S Nyman and J Lindhe, J Clin. Periodontal, 45 (1980) 725-730).

If, on the other hand, cement producing cells i.e. cementoblasts from the remaining adjacent and intact periodontal ligament tissue reach the denuded root surface area they will produce cementum with inserting connective tissue fibers on the dentine surface, i.e. a true, new periodontal ligament is formed uniting the root with the surrounding bone and gingival connective tissue. Unfortunately the cementoblasts do not normally reach more than a negligable part of the previously diseased root surface immediately adjacent to the intact periodontal ligament due to cells of the other tissues occupying the wound area. However, research into guided tissue regeneration has shown that the cementum and periodontal ligament producing cells have the ability to become established on the root surface if that surface is isolated from other tissues during healing (S. Nyman, J. Gottlow, T. Karring and J. Lindhe, J. Clin. Perio., 9 (1982) 257-265; S. Nyman, J. Lindhe, T. Karring and H. Rylander, J. Clin. Perio., 9 (1982) 290-296; J. Gottlow, S. Nyman, T. Karring and J. Lindhe, J. Clin. Perio., 11 (1984) 494-503). This isolation during the initial healing process enables the different periodontal structures to become reestablished in a proper sequence resulting in a new periodontal attachment.

It is known to use a biostable protective barrier of expanded polytetrafluoroethylene (PTFE) comprising a PTFE matrix of fine fibrils and containing open porosities (marketed under the registered trade mark GORE-TEX) between the gingival epithelium and connective tissue on one hand and the periodontal ligament and the alveolar bone on the other. The barrier creates a protected space adjacent to the periodontal wound so that cells from the remaining periodontal ligament can repopulate the root surface for periodontal regeneration however in competition with the cells of the alveolar bone. Thus the GORE-TEX periodontal material functions only in part selectively as long as the bone tissue is not separated from the periodontal ligament tissue during the healing process which means a risk for ankylosis formation.

In addition the GORE-TEX periodontal material often collapses into periodontal defects thus jeopardizing the objective of creating a space between the tooth and the material.

WO 86/02824 describes an element for controlling growth into surgically intervened areas, comprising a unitary element which forms cavities accessible for tissue growth through openings.

The biostable non-resorbable barriers tested so far must be removed after their function has been fulfilled, i.e. when the periodontal ligament cells have repopulated the denuded root surfaces. This means that a second operation must be performed. The removal operation is done typically 1 to 3 months after the first operation. This involves significant costs and additional risks (such as the possibility of postoperative infection) and troubles for the patient.

An ideal material for guided tissue regeneration should be biodegradable because such a material is resorbed in a biologic environment and, accordingly, no removal operation is necessary. However, attempts to utilize biodegradable barriers have failed due to disturbances of the healing process. For instance, well known natural non-biostable materials like collagen and catgut which are at least partially biodegradable, are not suitable for guided tissue regeneration because they induce rapidly (in 1 to 2 weeks) inflammatory reactions in living tissues (see e.g. E. Echeverria and J. Jiménez, Surgery, 131 (1970) 1-14 and J.B. Herrmann, R.J. Kelly and G.A. Higgins, Arch. Surg., 100 (1970) 486-490). The resorption of these materials is dependent upon the action of proteolytic enzymes (R.W. Postlethwait in "Davis-Christopher Textbook of Surgery" (D.C. Sabiston, Jr., ed.), 10th ed., Vol. I, Saunders Co., Philadelphia-London-Toronto, 1972, 307-318) which typically leads to early tissue reactions which interfere with the regeneration process of the periodontal tissues by retarding or preventing it. The biodegradable and non-biodegradable barriers utilized so far have neither been able to predictably differ regeneration of periodontal ligament tissue from regeneration of surrounding alveolar bone tissue nor have they been fully reliable as far as their space-making properties are concerned.

The object of the present invention is to provide a barrier which can be placed within the field of operation in such a way that a predictable healing sequence is achieved by the barrier predictably creating space for regeneration of, firstly, periodontal ligament tissue, secondly, surrounding alveolar bone tissue and, thirdly, gingival connective tissue and epithelium.

This object is achieved by a barrier of the kind referred to above which according to the invention has obtained the characteristics of claim 1.

The described healing sequence gives prerequisites for a predictable regeneration of all periodontal tissues surrounding the tooth instead of an incomplete repair of these tissues. In addition, in case of using a biodegradable material there is need for only one surgical procedure which is advantageous both with respect to costs and patients' comfort.

The barrier should preferably comprise a totally biodegradable material but could also comprise a partly biodegradable or a non-biodegradable material. The inventor has found that the material of the barrier should not be too reactive in its resorption behaviour and should retain its mechanical strength, at least partially, at least three weeks in vivo and during the first four weeks after implantation should induce only mild tissue reactions. Thus, the resorption of the barrier preferably should not start earlier than 4 to 6 weeks after the surgical implantation resulting in an initial period of healing free from resorptive disturbances. Also after the beginning of the resorption of the barrier the tissue effects should be minimal.

Suitable biodegradable materials comprise polymers such as polyglycolide (PGA), polylactide (PLA), stereocopolymers of PLA, and copolymers of PLA, and resorbable ceramic materials. Other examples are hyaluronic acids and mixtures of the mentioned materials. Still other examples are polydioxanon, polyhydroxy butyric acid, copolymers of polyhydroxy butyric acid and hydroxy valeric acid, and polyesters of succinic acid. Suitable non-biodegradable materials could be, but are not limited to, polyurethanes, polyesters, expanded polytetrafluorethylene and combinations of these materials.

The barriers should be very thin and preferably should have a maximum thickness of 200-300 µm.

The separating means of the barrier are preferably formed as protuberances which are preferably dimensioned to keep the barrier on a distance of 200-500-1000 µm from the surface of a tooth or implant. These spacers can however also be so long that they are able to prevent a barrier covering a bony defect, from collapsing thereinto.

In the cervical (tooth-neck) region a tight anchorage (retention) and bacteria tight sealing of the barrier against the root surface should be secured. This can be achieved by a perforation pattern in the cervical collar of the barrier comprising holes and tracks which can harbour suitable material for gluing the barrier to the crown or root surface by the aid of for instance resin.

The cervical portion of the material facing the gingival tissue is preferably characterized by preformed cavities which allow for ingrowth of connective tissue. The cavities could be designed as described in the Swedish patent No. 8405568-0. The cavities could advantageously also be included in the gingival portion of the whole barriers. The ingrowth of connective tissue into the preformed cavities as well as the adhesive connection of the collar to the tooth arrests epithelial migration coronal of the barrier.

The invention will be described in more detail below with reference to the accompanying drawings in which
FIG. 1 is a buccal side view (seen from the lip side) of a one-rooted tooth with a prior art barrier, the adjacent tissues being shown in vertical cross sectional view,
FIG. 2 is an enlarged fragmentary side view and cross sectional view showing the connection of the barrier of FIG. 1 to the root of the tooth,
FIG. 3 is a proximal (lateral) view of the tooth in FIGS. 1 and 2, the adjacent tissues being shown in vertical cross-sectional view.
FIGS. 4 to 6 are views similar to FIGS. 1 to 3 illustrating one embodiment of the invention,
FIGS. 7 to 9 are views similar to FIGS. 1 to 3 illustrating a second embodiment of the invention,
FIGS. 10 to 12 are views similar to FIGS. 1 to 3 illustrating a third embodiment of the invention,
FIGS. 13 and 14 are wiews similar to FIGS. 1 and 3 illustrating a fourth embodiment of the invention,
FIGS. 15 and 16 are views similar to FIGS. 1 and 3 illustrating a fifth embodiment of the invention,
FIGS. 17 and 18 are views similar to FIGS. 1 and 3 illustrating the same embodiment of the invention as in FIGS 7-9 applied, however, to a two-rooted tooth,
FIGS. 19 and 20 are views similar to FIGS. 17 and 18 illustrating the same embodiment of the invention as in FIGS 10-12 applied to a two-rooted tooth,
FIG 21 is a plan view of a blank for a barrier of sandwich structure,
FIG 22 is a side view of the barrier of sandwich structure obtained by using the blank of FIG 21,
FIG 23 is a view similar to FIG 12 of a modified barrier of sandwich structure,
FIG 24 is a plan view of the arrangement in FIG 22,
FIGS 25 and 26 are views similar to FIGS 7 and 9 of a still further embodiment of the invention,
FIGS. 27 and 28 are views similar to FIGS. 1 and 3 illustrating an embodiment of the invention applied to an artificial screw or similar implant,
FIGS. 29 and 30 are views similar to FIGS. 27 and 28 illustrating a second embodiment of the invention applied to an implant,
FIG 31 is a view similar to FIG 27 illustrating a third embodiment of the invention applied to an impland, and
FIG 32 is a view similar to FIG 27 illustrating a fourth embodiment of the invention applied to an implant.

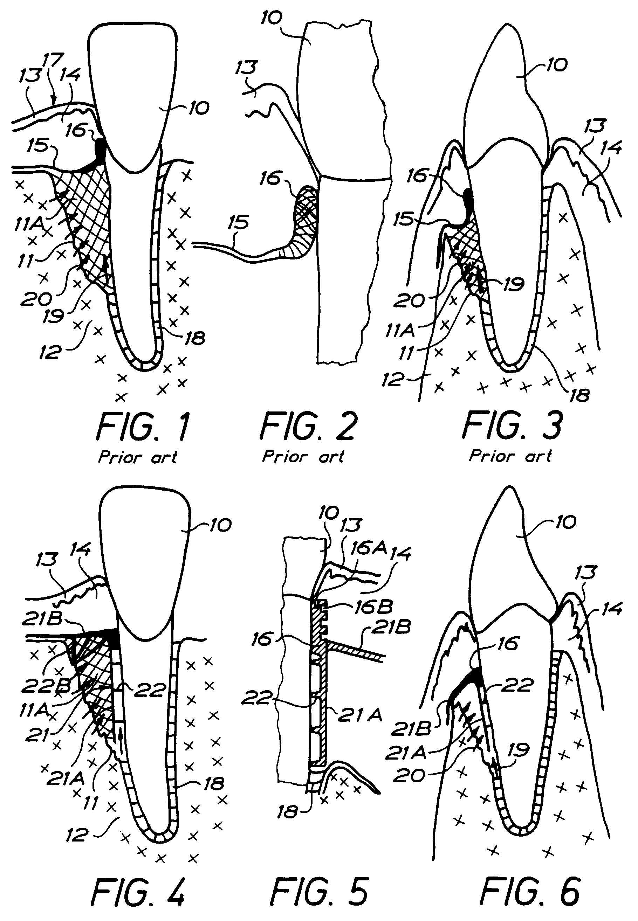

Referring to FIGS. 1 to 3 of the drawings, there is shown a one-rooted tooth 10 with a bony crater 11 in the bone tissue 12, extending over two of the root surfaces. Soft tissues, epithelium 13 and connective tissue 14, cover the bone tissue. The bony crater 11 has been debrided and is filled with a blood cloth 11A. A prior art barrier 15 has been placed in its position with a cervical portion 16 sutured around the neck of the tooth. The previously reflected soft (mucoperiosteal) flap 17 has been repositioned and covers the barrier. It is expected that the tissues which have been given preference, i.e. the periodontal ligament tissue 18 and the bone tissue 12 regenerate without being hindered by down-growing epithelium 13 and connective tissue 14 as indicated by a large arrow 19 and small arrows 20, respectively. There is, however, a risk that the bone tissue regeneration will be faster than the periodontal ligament cell regeneration thus preventing a regeneration of true periodontal ligament tissue along the root surface instead causing so-called ankylosis, i.e. bone tissue in direct contact with the root surface without intervening soft tissue. Such an arrangement has a considerable potential to cause more or less extensive resorptions of the root dentine ultimately destroying the whole root. This risk probably increases the narrower the bony crater, i.e. the shorter the distance between the bone wall and the root surface, but there are probably other, so far unknown factors that govern the relative regeneration velocity of these two types of tissue.

In order to fully eliminate the risk of "bone-to-root-healing" the barrier of the invention which permits full control over the healing sequences of these two tissues, can be used as is shown in FIGS. 4 to 6. These figures illustrate the same situation of tissue destruction as in FIGS. 1 to 3 but there is a decisive alteration in the design of the barrier generally designated 21, which has a first portion 21A close to the root surface but kept at a distance therefrom which can range from 10 µm to 1000 µm, preferably from 100 to 500 µm, by means of protuberances, bosses, or similar means forming spacers 22, and a second portion 21B extending transversely from the root at the upper end of portion 21A. Also this portion preferably provides spacers 22B reaching from the barrier portion 21B to the bone wall in order to eliminate the risk of barrier collapse in case of wide defects as the barrier itself must be rather soft to be adequately shaped. The spacers accordingly should be relatively long and stiff when used to support barriers protecting wide defects or forming other tent-like housings, and the necessary stiffness can be imparted to them by reinforcing the spacers or by forming the spacers of another material than that the barrier is made of, or by including into the spacers the same mixture of materials as in the barrier itself but in another ratio. Thus, the spacers can comprise a combination of materials of biodegradable and/or biostable type.

The barrier is advantageously porous or at least partially porous or microporous.

A collar portion 16 is glued to the tooth root and additionally is sutured to the root as shown at 16A. The collar portion forms undercut cavities 16B preferably designed as described in the Swedish patent No. 8405568-0 as bottom holes or grooves.

A porous barrier can be manufactured essentially of fibers by knitting, weaving or by non-woven techniques. Such fibrous materials or devices can contain biodegradable polymeric material as fiber binding material, or the fibers can be bound to each other only by means of physical forces.

Also biodegradable ceramic fibers, including calciumphosphate or hydroxyapatite fibers, can be used.

To help the spacers in preventing collapse of the barrier when protecting wide defects the barrier could also be provided with local fiber reinforcements in the form of stroma not jeopardizing the malleability of the barrier.

Clinical observations have indicated that with a barrier including the biodegradable materials mentioned above sufficient healing may occur between 4-6 weeks. Up to 4 weeks the materials cause only slight cellular response and therefore they behave during these four first weeks much in the same manner as biostable inert barriers (such as polytetrafluoroethylene). After four weeks the healing of periodontal tissues has proceeded significantly and the resorption of the barrier can accelerate without significantly disturbing the regenerated periodontal tissues. Because the material when biodegradable disintegrates and disappears by hydrolysis no removal operation is later necessary.

The space between the portion 21A associated with the root surface, and the bone wall, i.e. the bony crater 11, can also be more or less occupied by a three-dimensional mesh consisting of biodegradable threads of one of the materials for the barrier mentioned above, which should make easier the regeneration of bone.

The material of the barrier and/or the mesh can contain also different additives because of different purposes to facilitate the processing, to modify the properties, to help the surgical installation or to give to the material and/or device totally new biofunctional properties. Such additives are e.g. colours, plasticizers, antioxydants and biofunctional additives such as drugs (e.g. antibiotics), growth hormones, etc. Thus, the threads of the mesh can be loaded with slowly released bone growth stimulating components as can also the parts of the barrier facing the bony crater. The spacers and the side of the barrier portion 21 facing the root surface preferably are loaded with slowly releasing components stimulating the regeneration of periodontal ligament tissue. The surface facing the gingival tissue, in particular the collar portion, can be loaded with slowly released components stimulating the ingrowth of gingival tissue into the material. The threads and the barrier itself in case of being loaded with such components are constructed to either physically (micro-cavities) and/or chemically bind the different components. Suitable components to be used for loading are protein components to stimulate bone regeneration and enamel matrix components e.g. from pigs or bovine to stimulate periodontal tissue regeneration, and growth hormones such as TGF-alfa, TGF-beta, EGF, PDGF, FGF and IGF-1. The design of this type of barrier thus permits not only a discrimination of the two different tissues but also a predictable determination of the amount of regeneration tissue due to the size of the spacers and an individual shaping of the marginal portion of the barrier. In addition, the size of the barrier holes can predictably be varied from less than 1 um up to about 100 µm or more, thereby governing the transportation of tissue fluid components as well as the transmigration possibilities or velocity of the tissue cells responsible for the rebuilding of the different types of tissue.

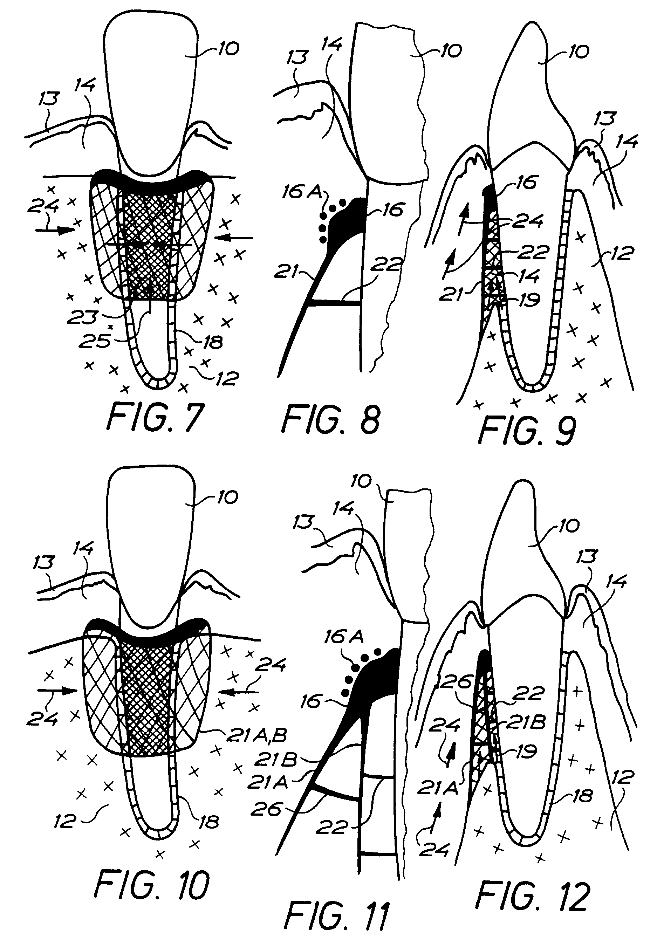

FIGS. 7 to 9 show a one-rooted tooth from the same aspects as in FIGS. 4 to 6 but with a denuded buccal root surface indicated at 23 in FIG. 7 by shading. The barrier 21 is placed to cover the denuded root surface which has been thoroughly cleaned after that a mucoperiosteal flap has been raised to uncover it and the surrounding alveolar bone 12. The barrier also covers the bone adjacent to the denuded root surface. The cervical portion 16 of the barrier is glued to the root surface and extra-long spacers 22 define the space between barrier 21 and the root surface. This space is occupied by a blood cloth 14 after that the mucoperiosteal flap has been mobilized in coronal or lateral direction (towards the crown of the tooth or from either side of the root, respectively, as indicated by large arrows 24) and sutured tight to the tooth in its new position at 16A. It is expected that the favourized tissues, i.e. periodontal ligament and bone tissue (small arrows) to a considerable degree will regenerate in coronal direction and from the lateral regions as indicated by small arrows 25, to cover the denuded root surface. The barrier, however, does not discriminate between periodontal ligament tissue and bone tissue with risk of ankylosis due to faster bone regeneration than periodontal ligament regeneration. The barrier design in FIGS. 10 to 12 eliminates such a risk.

FIGS. 10 to 12 illustrate the same anatomical situation as in FIGS. 7 to 9 but with a barrier which discriminates between the bone regeneration and the periodontal ligament regeneration as illustrated in the proximal view of FIG. 12. Extra spacers 26 are provided between an outer barrier portion 21A and an inner barrier portion 21B to maintain the space for bone regeneration. This space can preferably be supplied with threads to stimulate the bone regeneration as previously discussed.

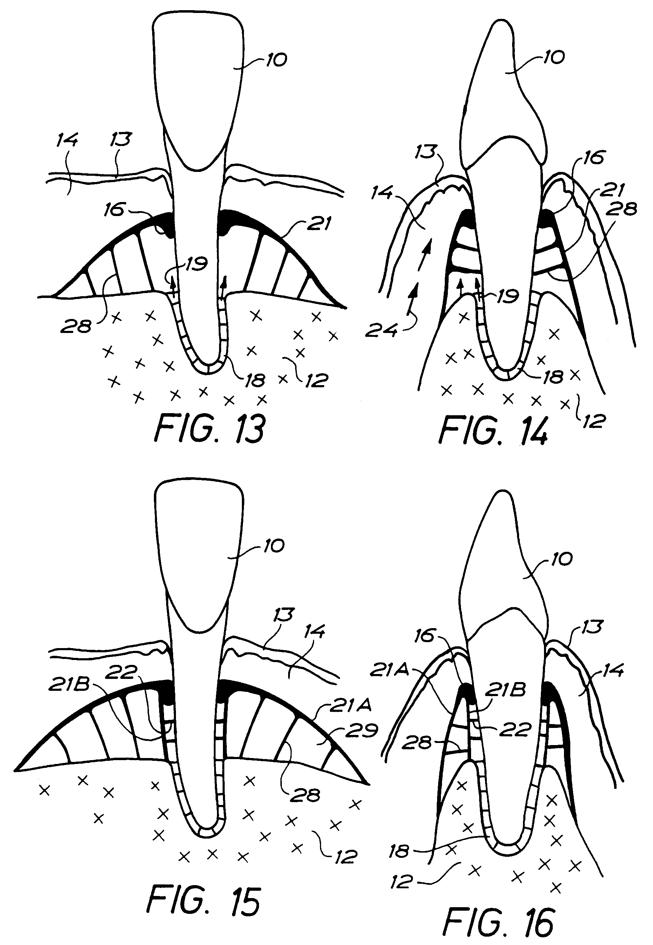

FIGS. 13 and 14 show the same aspects as in FIGS. 1 to 12 but with so-called horizontal bone destruction, i.e. the destruction of bone is equal all around the root with no bony craters surrounded by bone walls. The barrier is basically constructed as illustrated in FIGS. 7 to 9 and comprises a single barrier layer 21 but with specially designed spacers 28 reaching from the barrier to the root surface or the bone. These spacers at the same time can serve as guiding structures for regeneration of the bone tissue in coronal direction (in direction of the tooth crown). The stiffness of this type of barrier is adjusted to withstand the pressure from the soft tissues. This is done during the production procedure by mixing the barrier material components in certain ratiosor by reinforcing the spacers and the barrier by other materials. Also this type of barrier cannot, however, predictably discriminate between the bone and the periodontal tissue regeneration. A discriminating barrier suitable for this type of bone destruction is shown in FIGS. 15 and 16.

FIGS. 15 and 16 show the same bone destruction situation as in FIGS. 13 and 14. However, the barrier again has two portions, an outer portion 21A intended to separate the connective tissue 14 from a space 29 into which the bone tissue 12 should regenerate, and an inner portion 21B separating this space from the space adjacent to the root surface into which the periodontal ligament tissue 18 should regenerate. The barrier portions 21A and 21B are held in the proper positions by spacers 28 and 22, respectively. Thus, the risk of causing ankylosis, i.e. bone regeneration in close contact with the root surface, is eliminated.

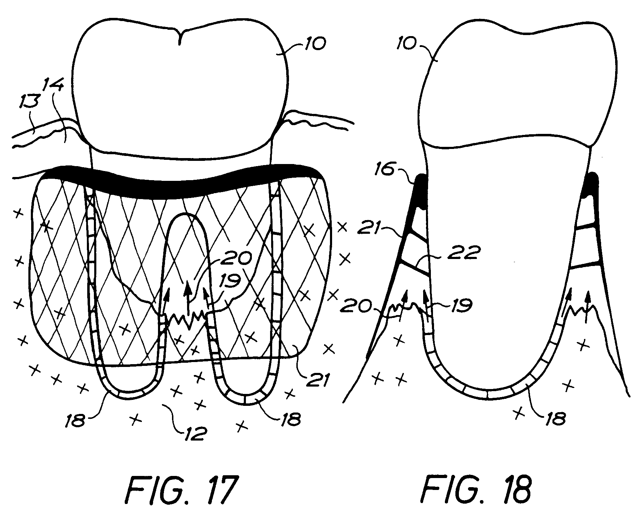

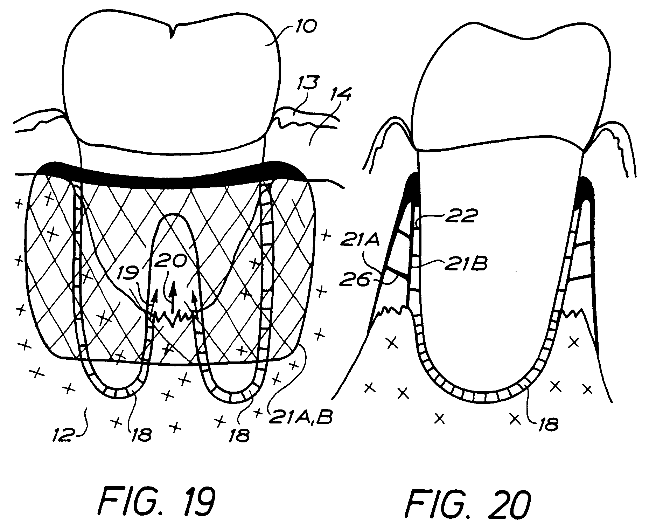

FIGS. 17 and 18 show a two-rooted tooth with a so-called bifurcation defect, i.e. a destruction of the tooth-supporting tissue (periodontal ligament and bone) between the two roots of the tooth. The barrier 21 is applied to cover the entrance of the bifurcation either on the buccal side or on the lingual side or on both. The spacers 22 maintain the space between the root surfaces of the two roots and the barrier. The barrier is glued at the cervical portion 16 to the cervical parts of the roots as previously discussed. Also in this case the one-layer type of barrier presented in these figures does not secure a predictable regeneration concerning the bone in relation to the periodontal ligament. It is therefore a certain risk of undesirable bone regeneration in direct contact with the root surface (ankylosis). To make sure that such healing is avoided the barrier can be modified according to FIGS. 19 and 20 which illustrate the same type of tissue destruction as in FIGS. 17 and 18 but with a barrier that has an outer portion 21A and an inner portion 21B. Said latter portion is close to the surface of each of the two roots on a predictable distance from that surface obtained by spacers 22. The barrier portion 21B is connected to the outer barrier portion 21A by means of other spacers 26 which keep the two barrier portions in a predetermined relation to each other. These structures can also serve as a skeleton for the regeneration of bone cells and bone tissue. An alternative is to use threads forming a three-dimensional mesh as a scaffold for the regenerating tissue.

It should be noted that one or the other layer of the double-layer barriers described above can be used alone depending on the location and character of the defect to be healed.

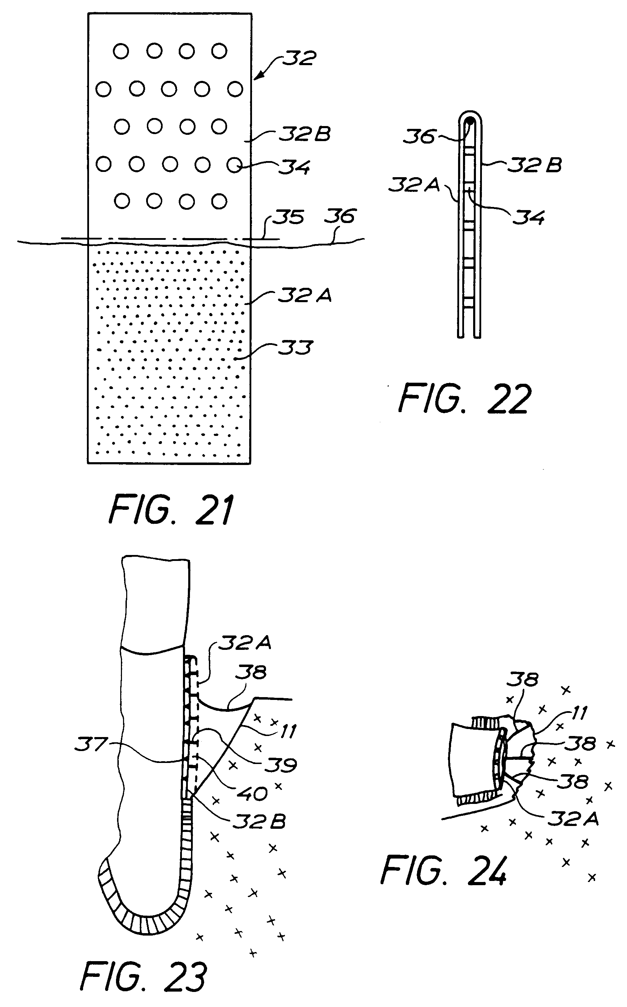

FIGS. 21 and 22 disclose a barrier of the invention which forms a sandwich structure. The barrier is made of a blank 32 having two substantially equal portions 32A and 32B, which is a foil having a thickness e.g. of about 100 µm. Portion 32A is perforated having apertures 33 of a diameter e.g. of about 200 µm spaced e.g. about 400 µm from centre to centre, or corresponding slots. Portion 32B forms cylindrical protuberances 34 e.g. of a diameter about 400 µm and a height of about 200 µm, the space between the protuberances being e.g. 1.27 mm. The two portions have been folded along the dividing line 35 between the two portions with the protuberances of portion 32B abutting portion 32A, said protuberances forming spacers between the two portions which accordingly are mutually spaced about 200 µm. The two portions 32A and 32B are attached to each other by the end surfaces of the protuberances 34 being glued or otherwise connected to portion 32A. A suture 36 can be located in the fold to be used for attaching the sandwich structure to a tooth, but such structure can also be glued to the tooth. The sandwich structure should be attached to the tooth with portion 32B facing the tooth.

A further development of the sandwich barrier of FIGS. 21 and 22 is disclosed in FIGS. 23 and 24. In these figures portion 32B has spacers 37 at the side thereof which faces the tooth. Moreover, it is shown that the barrier is pressed against the tooth by means of anchoring pins 38 which have some springiness so that they can be pressed into the bone crater 11 to span under tension the distance between the barrier and the crater wall. These pins may be replaced by a wedge or a body filling the bone crater. In the embodiment of FIGS. 23 and 24 the protuberances of FIGS. 21 and 22 are replaced by ribs 39 extending substantially in the horizontal direction so that the space between portions 32A and 32B of the sandwich structure is divided into a number of individual compartments 40 forming undercut cavities, to which the surrounding tissue has access through the apertures 33.

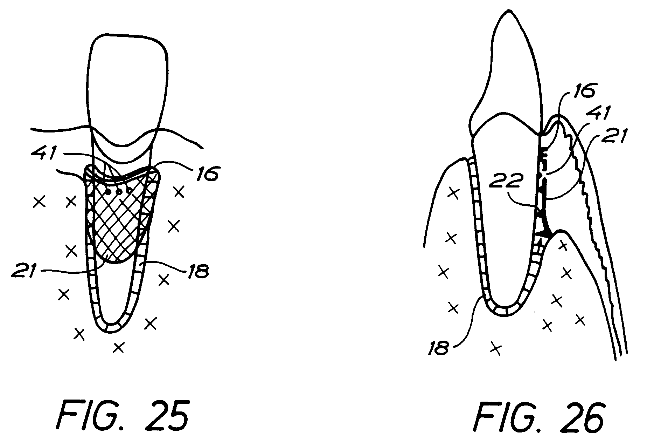

In FIGS. 25 and 26 there is disclosed a single barrier 21 of the type shown in FIG. 9. This barrier has spacers 22 at the side thereof facing the tooth, and it has a cervical portion 16 which is attached to the tooth. The barrier is impervious except that there are provided in the upper portion thereof adjacent the cervical portion 16 three central apertures 41, which thus are remote from the side edges of the barrier and also from the front of the peridontal ligament tissue 18. These apertures should be dimensioned such that the penetration of cells from surrounding tissue is retarded or delayed but an unobstructed growth of tissue through the barrier is prevented since this would spoil the purpose of the barrier. The apertures allow the supply of oxygene and nutrition to the space between the barrier and the tooth, which may stimulate the growth of cervical tissue into said space so that the space will be substantially filled by such tissue before growth of surrounding tissue into the space between the barrier and the tooth through the apertures has started.

Apertures of the type mentioned above may also be provided in a barrier such as barrier portion 21B in FIG. 6. In that case the apertures should be located near the wall of the bone crater.

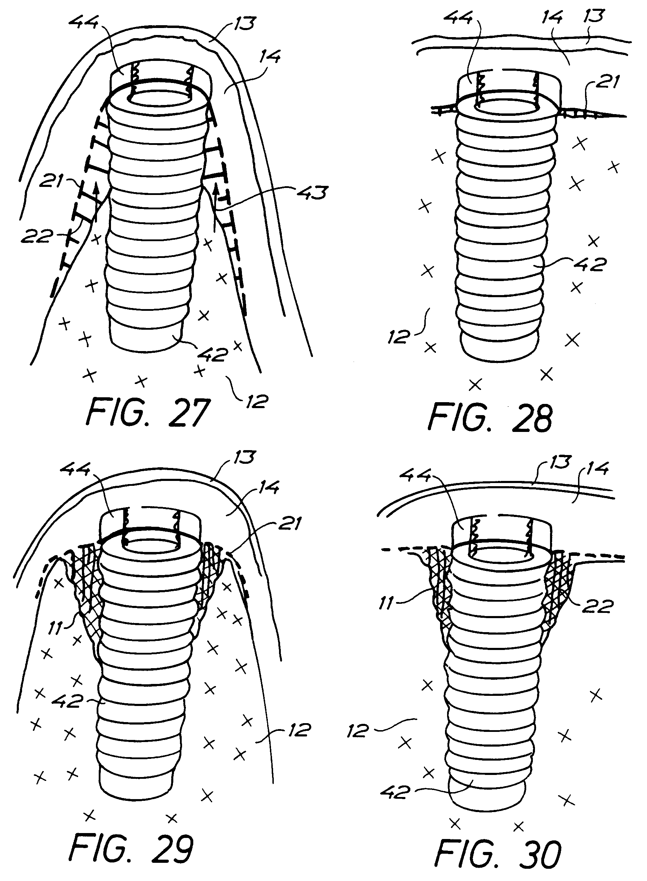

FIGS. 27 and 28 show a cylindrical implant 42 of a biocompatible material. The implant is shown to be of the screw type but can be of any other type known in the art. The implant is installed in the bone 12 but as shown in FIG. 27 the bone covers only the lower half of the implant while the rest of the implant is covered by connective tissue 14 and epithelium 13. A biodegradable or non-biodegradable barrier 21 covers the upper half of the implant and parts of the bone tissue 12. Spacers 22 maintain a predetermined space for the bone tissue cells to regenerate in superficial direction as indicated by arrows 43. The bone surface facing the space is preferably mechanically traumatized to cause bleeding and expose the subsurface to facilitate the bone growth into the space. The spacers 22 comprise protuberances or bosses on the barrier but can also comprise threads forming a mesh-like structure serving also as a scaffold for regenerating bone cells and other components. The barrier components preferably are loaded with bone stimulating preparations on the side of the barrier facing the space as well as on the spacers or the mesh, respectively. The barrier is retained to the implant using a specially designed screw 44.

FIGS. 29 and 30 illustrate the application of the same type of barrier as in FIGS. 27 and 28 but with the barrier covering a bony crater 11 which is defined between the implant wall and the alveolar bone, the barrier covering the upper (superficial) entrance of the crater.

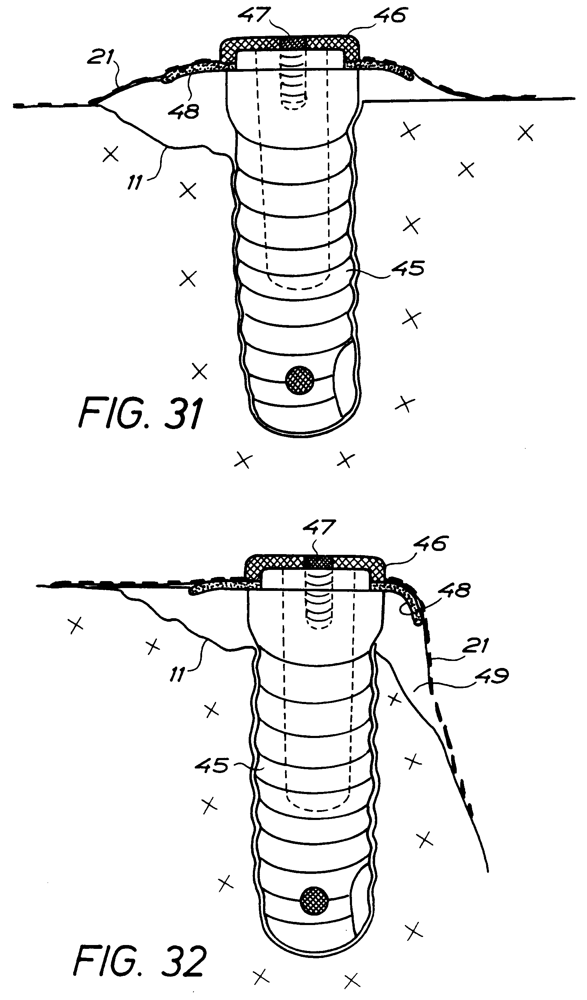

FIGS. 31 and 32 disclose further embodiments of the invention in connection with an implant 45. In these embodiments the barrier 21 is clamped between a cap 46, which is secured to the implant end surface by means of a screw 47 screwed in the implant, together with an underlying element 48, which may be formed as a disc, a star or a spider. This element is stiff enough to support the barrier, so that it will be held spaced from the bone 12 covering a bone crater 11. Element 48 may be made of metal or plastic material which can be plastically deformed at room temperature or at an elevated temeperature so that it may be adapted to the desired form of the barrier as shown to the right in FIG. 32 wherein element 48 has been bent down so that the barrier extends substantially vertically to cover a space 49 to be restored by bone tissue growth thereinto.

The barrier of the invention can be constructed in other ways than those described with reference to the drawings. It is also possible to provide barriers of any type with active substances of the kind referred to herein.

## Claims

1. Barrier for guided or controlled tissue regeneration, comprising a shapable thin membrane, **characterized** by spacer means projecting from the surface of the membrane on at least one side thereof so that said one side is separable from adjacent tissue or an adjacent second barrier by said spacer means.

2. Barrier as claimed in claim 1, **characterized** in that the barrier at least partially comprises a biodegradable material.

3. Barrier as claimed in claim 1, **characterized** in that the barrier at least partially comprises a non-biodegradable material.

4. Barrier as claimed in claim 1, **characterized** in that said spacer means comprise protuberances on said one side of the barrier.

5. Barrier as claimed in claim 4, **characterized** in that the protuberances are tapered towards the free end thereof.

6. Barrier as claimed in claim 1, **characterized** in that said spacer means comprise a macromolecular matrix on said one side of the membrane, e.g. hyaluronic acid.

7. Barrier as claimed in any of claims 1 to 6 **characterized** in that the barrier comprises two portions folded against each other said spacer means separating one portion from the other portion.

8. Barrier as claimed in any of claims 1 to 7, **characterized** in that said spacer means define a space ranging from 5 to 5000 µm, preferably from 100 to 500 µm.

9. Barrier as claimed in any of claims 1 to 8, **characterized** in that the barrier is at least partially porous or perforated.

10. Barrier as claimed in claim 2, **characterized** in that it is manufactured at least partially of biodegradable polymer, copolymer, polymer alloy, polydioxanon, polyhydroxy butyric acid, copolymers of polyhydroxy butyric acid and hydroxy valeric acid, and polyesters of succinic acid, or a mixture or composite thereof.

11. Barrier as claimed in claim 3 **characterized** in that it is manufactured at least partially of non-biodegradable polymer, polyurethane, polytetrafluoroethylene or polyester, or a mixture or composite thereof.

12. Barrier as claimed in claim 10, **characterized** in that it is manufactured of at least one of the polymers in the group comprising: polyglycolides (PGA), polylactides (PLA), and glycolide/lactide copolymers (PGA/PLA),

13. Barrier as claimed in claim 4 **characterized** in that the protuberances are manufactured of or reinforced with biodegradable and/or biostable fibers.

14. Barrier as claimed in any of claims 1 to 12, **characterized** in that the barrier contains a biofunctional active substance such as peptide matrix from gingival connective tissues, periodontal ligament tissues and enamel tooth organs, antiseptica, antibiotica, growth hormones, or other drugs.

15. Barrier as claimed in claim 13, **characterized** in that said substance comprises a peptide, such as fibronectin or vitronectin, or a growth hormone in the group comprising TGF-alfa, TGF-beta, EGF, PDGF, FGF and IGF-1.

16. Barrier as claimed in any of claims 1-15, **characterized** in that the barrier forms a passage and that a suture (36) extends through said passage.

## Patentansprüche

1. Grenzschicht für geführte oder kontrollierte Geweberegeneration, umfassend eine formbare dünne Membran, gekennzeichnet durch von der Oberfläche der Membran an mindestens einer Seite derselben derart abstehende Abstandhaltermittel, daß diese eine Seite durch die Abstandhaltermittel von angrenzendem Gewebe oder einer angrenzenden zweiten Grenzschicht trennbar ist.

2. Grenzschicht nach Anspruch 1, dadurch gekennzeichnet, daß sie zumindest teilweise aus einem biologisch abbaubaren Material besteht.

3. Grenzschicht nach Anspruch 1, dadurch gekennzeichnet, daß sie zumindest teilweise aus einem nicht biologisch abbaubaren Material besteht.

4. Grenzschicht nach Anspruch 1, dadurch gekennzeichnet, daß die Abstandhaltermittel aus an der einen Seite der Grenzschicht vorgesehenen Vorsprünge bestehen.

5. Grenzschicht nach Anspruch 4, dadurch gekennzeichnet, daß die Vorsprünge sich in Richtung auf ihre freien Enden verjüngen.

6. Grenzschicht nach Anspruch 1, dadurch gekennzeichnet, daß die Abstandhaltermittel eine makromolekulare Matrix an der einen Seite der Membran, z.B. (aus) Hyaluronsäure, umfassen.

7. Grenzschicht nach einem der Ansprüche 1 bis 6, dadurch gekennzeichnet, daß sie zwei gegeneinander gefaltete Abschnitte oder Bereiche aufweist, wobei die Abstandhaltermittel den einen Bereich vom anderen Bereich trennen.

8. Grenzschicht nach einem der Ansprüche 1 bis 7, dadurch gekennzeichnet, daß die Abstandhaltermittel einen (Zwischen-)Raum im Größen-Bereich von 5 - 5000 µm, vorzugsweise 100 - 500 µm, festlegen.

9. Grenzschicht nach einem der Ansprüche 1 bis 8, dadurch gekennzeichnet, daß sie zumindest teilweise porös oder perforiert ist.

10. Grenzschicht nach Anspruch 2, dadurch gekennzeichnet, daß sie zumindest teilweise aus einem (einer) biologisch abbaubaren Polymeren, Copolymeren, Polymerlegierung, Polydioxanon, Polyhydroxybuttersäure, Copolymeren von Polyhydroxybuttersäure und Hydroxyvaleriansäure sowie Polyestern von Bernsteinsäure oder einem Gemisch oder Verbundstoff davon hergestellt ist.

11. Grenzschicht nach Anspruch 3, dadurch gekennzeichnet, daß sie zumindest teilweise aus einem nicht biologisch abbaubaren Polymeren, Polyurethan, Polytetrafluorethylen oder Polyester oder einem Gemisch oder Verbundstoff davon hergestellt ist.

12. Grenzschicht nach Anspruch 10, dadurch gekennzeichnet, daß sie aus mindestens einem Polymeren aus der Gruppe Polyglycolide (PGA), Polylactide (PLA) und Glycolid/Lactid-Copolymeren (PGA/PLA) hergestellt ist.

13. Grenzschicht nach Anspruch 4, dadurch gekennzeichnet, daß die Vorsprünge aus biologisch abbaubaren und/oder biostabilen Fasern geformt oder mit diesen versteift sind.

14. Grenzschicht nach einem der Ansprüche 1 bis 12, dadurch gekennzeichnet, daß sie eine biofunktionelle aktive Substanz, wie Peptidmatrix von Zahnfleischbindegeweben, periodontalen Ligamentgewebe und Zahnschmelz-Zahnorgane, Antiseptika, Antibiotika, Wachstumshormone und andere Medikamente, enthält.

15. Grenzschicht nach Anspruch 13, dadurch gekennzeichnet, daß die Substanz ein Peptid, wie Fibronectin oder Vitronectin, oder ein Wachstumshormon aus der Gruppe TGF-alfa, TGF-beta, EGF, PDGF, FGF und IGF-1 umfaßt.

16. Grenzschicht nach einem der Ansprüche 1 bis 15, dadurch gekennzeichnet, daß sie einen Durchgang bildet und daß ein Näh-Faden (36) durch den Durchgang verläuft.

## Revendications

1. Barrière pour la régenération guidée ou contrôlée de tissus, comprenant une fine membrane façonnable, caractérisée par des moyens d'écartement qui font saillie sur la surface de la membrane d'un cote au moins de celle-ci, de sorte que ce cote puisse être séparé de tissus voisins ou d'une seconde barrière voisine par lesdits moyens d'écartement.

2. Barrière selon la revendication 1, caractérisée en ce que la barrière est faite au moins en partie d'une matière biodégradable.

3. Barrière selon la revendication 1, caractérisée en ce que la barrière est faite au moins en partie d'une matière non biodégradable.

4. Barrière selon la revendication 1, caractérisée en ce que lesdits moyens d'écartement sont constitues par ces protubérances sur ledit cote de la barrière.

5. Barrière selon la revendication 4, caractérisée en ce que les protubérances s'amincissent en direction de leur extrémite libre.

6. Barrière selon la revendication 1, caractérisée en ce que lesdits moyens d'écartement sont constitues par une matrice macromoléculaire sur ledit cote de la membrane, par exemple en acide hyaluronique.

7. Barrière selon l'une quelconque des revendications 1 à 6, caractérisée en ce que la barrière comprend deux parties repliées l'une contre l'autre, lesdits moyens d'écartement séparant l'une des parties de l'autre.

8. Barrière selon l'une quelconque des revendications 1 à 7, caractérisée en ce que lesdits moyens d'écartement définissent un espace de 5 à 5000 µm, de préférence de 100 à 500 µm.

9. Barrière selon l'une quelconque des revendications 1 à 8, caractérisée en ce que la barrière est au moins partiellement poreuse ou perforée.

10. Barrière selon la revendication 2, caractérisée en ce qu'elle est fabriquée au moins en partie en polymére, copolymere ou ailiage de polyméres biodegradable, en polydioxanone, en acide polyhydroxybutyrique, en copolyméres d'acide polyhydroxybutyrique et d'acide hydroxyvalerique, en polyesters d'acide succinique ou en un mélange ou produit composite de ces matières.

11. Barrière selon la revendication 3, caractérisée en ce qu'elle est fabriquée au moins partiellement en polymére non biodegradable, en polyurethanne, en polytetrafluoroéthyléne, en polyester ou en un mélange ou produit composite de ces matières.

12. Barrière selon la revendication 10, caractérisée en ce qu'elle est fabriquée en au moins l'un des polyméres du groupe constitue par les polyglycolides (PGA), les polylactides (PLA) et les copolymères glycolide/lactide (PGA/PLA).

13. Barrière selon la revendication 4, caractérisée en ce que les proturbérances sont fabriquées en fibres biodegradables et/ou biostables ou sont renforcées avec de telles fibres.

14. Barrière selon l'une quelconque des revendications 1 à 12, caractérisée en ce que la barrière contient une substance active biofonctionnelle, telle qu'une matrice peptidique provenant de tissus conjonctifs gingivaux, de tissus du ligament periodontique et d'organes dentaires a émail, des antiseptiques, des antibiotiques, des hormones de croissance ou d'autres médicaments.

15. Barrière selon la revendication 14, caractérisée en ce que ladite substance est constituée par un peptide tel que la fibronectine ou la vitronectine, ou par une hormone de croissance du groupe comprenant TGFα, TGFβ, EGF, PDGF, FGF et IGF-1.

16. Barrière selon l'une quelconque des revendications 1 à 15, caractérisée en ce qu'un passage est forme dans la barrière et en ce qu'un fil de suture (36) s'étend à travers ce passage.
